# EUROPEAN PATENT APPLICATION

(11) **EP 4 599 802 A1**
(43) Date of publication of application: **13.08.2025**
(21) Application number: 25156361.5
(22) Date of filing: 06.02.2025
(51) Int. Cl.: A61F 2/30, A61F 2/38

(54) **DEVICE AND METHOD FOR MAKING A TIBIAL SPACER**

(30) Priority: 08.02.2024 IT 202400002629
(71) Applicant: G21 S.r.l., 41039 San Possidonio (MO) (IT)
(72) Inventor: FORONI, Filippo, 41037 Mirandola (Modena) (IT)
(74) Representative: Gagliardelli, Fabrizio

(57) **Abstract**

Described is a device (1) for making a tibial spacer (10) for a temporary knee prosthesis made of medical cement, comprising: a tibial plate module (2), defining a first moulding chamber (MC1) configured for making a tibial plate (20),
a stem module (3), defining a second moulding chamber (MC2) configured for making at least partly a tibial stem (30) designed to be inserted in a tibial channel;
wherein the first moulding chamber (MC1) and the second moulding chamber (MC2) are fluid dynamically communicating, to allow a tibial spacer (10) to be made in a single body.

Described is an assembly (100) for making a tibial spacer (10) of a temporary knee prosthesis comprising the device (1) and a reinforcing core (40) which can be positioned at least partly inside the second moulding chamber (MC2) of the device (1).

## Description

This invention relates to the field of revision surgery and, in particular, of revision surgery following the failure of a knee prosthesis.

The object of this invention is, in particular, a module for making a tibial spacer in an operating room, which can be used in the surgical procedures for revising the knee joint; further objects of this invention are a method for making a tibial spacer starting from said module and the tibial spacer made according to said method.

The term spacer means a medical device made of medical cement, that is, of bone cement impregnated with antibiotic, designed for implantation in place of an infected joint prosthesis, in order to temporarily restore the functionality of the implant seat and treat the active infection in said implant seat.

The spacers are used during surgical protocols, known as "revisions", in two steps, wherein a first step comprises the explanation of the infected prosthesis and its replacement with the corresponding spacer, and wherein a second step, when the infection is completely treated, comprises the removal of the spacer and the insertion of a new joint prosthesis.

The knee joint spacers comprise a femoral component, called femoral spacer, and a tibial component, called tibial spacer, which are configured to articulate so as to restore, as much as possible, the physiological functionality of the knee joint, at the femoral condyles and the tibial plateau, respectively.

The tibial spacer and the femoral spacer are obtained by moulding medical cement in an operating room inside, respectively, a femoral module and a tibial module, an operation which is performed by an orthopaedic surgeon at the same time as the explanation of the infected prosthesis.

Currently, in order to improve the stabilisation of the spacer, the surgeon can make, in an operating room, a stabilising element made of medical cement, called stem or "taproot", provided with an elongated shape and configured to be inserted in a region of the tibia called tibial channel; the tibial channel extends inside the tibia from the proximal end to the distal end and is characterised by the presence of spongy tissue, through which the stem can be easily tucked in during the implantation.

Typically, said stem is obtained by manually modelling, in the operating room, a quantity of medical cement to form an elongated body which, once solidified, is cemented to the tibial component of the spacer and positioned proximal to the tibia, inserting the stem inside the tibial channel. Alternatively, the above-mentioned stem may be preformed, that is to say, it can be made before the revision operation, for example, by moulding or by using other methods known to experts in the sector; the preformed stem is therefore made available for the subsequent use: during the operation, said preformed stem is fixed by the surgeon to the tibial component, made in the operating room, using as an adhesive a predetermined quantity of medical cement and obtaining the tibial spacer to be implanted proximal to the tibia of the patient.

The tibial spacer comprising the stem can be more stably constrained to the tibia, compared with other prior art spacers.

However, the prior art solutions, both shaped by hand and preformed, have the limitation of not being able to guarantee a constant quality in the making of the tibial spacers; the effectiveness of the tibial spacer, consequently, depends on the individual ability of the surgeon who makes the stem manually or who cements the preformed stem to the remaining tibial component, and on the specific conditions of the operation.

The technical purpose of the invention is therefore to provide a device, a kit and a method for making a tibial spacer in an operating room which are able to overcome the obvious limitations of the prior art. The technical purpose of the invention is also to provide a tibial spacer made in an operating room using said device and kit and following said method.

The technical purpose and the aims specified are achieved by a device and a kit for making a tibial spacer in an operating room, a relative method and the tibial spacer made by means of said device and said kit and by said method, comprising the technical features described in one or more of the appended claims.

Further features and advantages of the invention are more apparent in the non-limiting description which follows of a preferred non-limiting embodiment of a device and an assembly for making a tibial spacer in an operating room, a relative method for making it and the tibial spacer made by means of said device and by means of said method.

The description is set out below with reference to the accompanying drawings which are provided solely for purposes of illustration without restricting the scope of the invention and in which:
- Figure 1 shows an isometric view of an assembly comprising the device for making a tibial spacer according to the invention;
- Figure 2 shows a partly exploded isometric view of the assembly of Figure 1;
- Figure 3 shows an exploded view of a detail of the assembly of Figure 2;
- Figure 4a shows an isometric view of a reinforcing core for a tibial spacer included in the assembly according to this invention.
- Figure 4b shows a side view of the reinforcing core of Figure 4a;
- Figure 5 shows an isometric view of a stem module included in the device for making a tibial spacer according to this invention;
- Figure 6 shows a first embodiment of a tibial spacer obtained using the device according to this invention;
- Figure 7 shows a second embodiment of a tibial spacer obtained using the device according to this invention;
- Figure 8 shows an isometric view from below of a half-module for the stem included in the device for making a tibial spacer of Figures 1-3 and Figure 5, according to an alternative embodiment;
- Figure 9 shows a side view of a modular reinforcing core included in the assembly according to this invention.

The first object of this invention is a device 1 for making a tibial spacer 10 in a single body having a tibial plate 20 and a tibial stem 30.

Preferably, but not exclusively, said tibial spacer 10 is made of medical cement, with the addition of an active compound, such as an antibiotic drug for the treatment of infections which have arisen at the implant site.

The device 1 according to the invention comprises a tibial plate module 2 and a stem module 3, preferably distinct and separate from the tibial plate module 2. Preferably, the two modules 2, 3 can be coupled in a removable fashion. Versions of the invention are not excluded which are defined in a different manner, for example they are not or are not completely separate or, for example, their coupling is not removable.

The following is a detailed description of the device 1 according to this invention; the term "proximal" will be used in the description to indicate elements or parts of elements which, in use, are closer to the femoral bone or devices which make said elements or parts of elements closer to the femoral bone, while the term "distal" will be used to indicate elements or parts of elements which, in use, are further from the femoral bone or devices which make said elements or parts of elements further from the femoral bone.

The tibial plate module 2 defines a first moulding chamber MC1 for making the tibial plate 20. More in detail, the first chamber MC1 is an inner space of this module 2 and is defined by the shape of a relative inner surface. The first moulding chamber MC1 is configured for receiving a quantity of medical cement in a liquid form and for allowing the solidification to obtain the tibial plate 20, which in use is configured for restoring the functionality of the proximal part of the tibia and, in particular, of the proximal tibial epiphysis. The first moulding chamber MC1 is, therefore, internally shaped to reproduce during moulding the anatomy of a proximal tibial epiphysis, that is, the tibial plate, that is to say, the portion of tibia which joins with the femur and, in particular, with the distal femoral epiphysis. More in detail, the first moulding chamber MC1 has a proximal surface and a distal surface opposite and connected by a lateral surface, defining the thickness of said first moulding chamber MC1. The distal surface is substantially flat, whilst the proximal surface has, preferably, two substantially flat or rounded lateral portions for making, respectively, a medial tibial condyle and a lateral tibial condyle in the tibial spacer 10; moreover, preferably, the proximal surface of the first moulding chamber MC1 has a convex central portion between the two lateral portions for making an intercondylar eminence, to obtain a tibial spacer 10 having a tibial plate 20 with a three-dimensional shape reproducing the anatomy of the proximal tibial epiphysis.

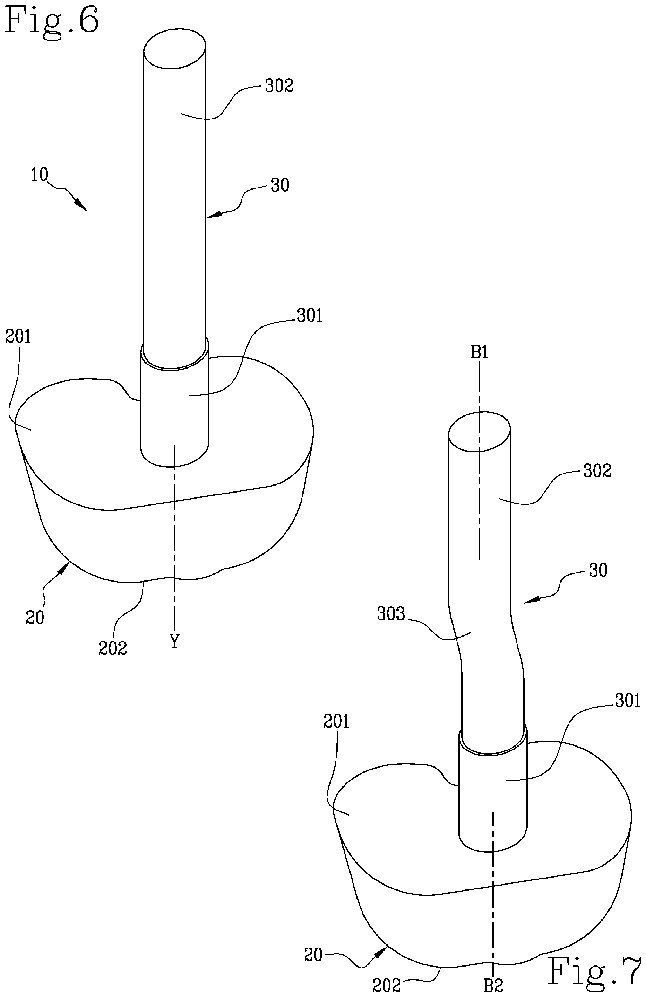

With reference to Figure 7, the distal surface of the first moulding chamber MC1 forms a lower surface 201 of the tibial plate 20, which in use is positioned at the tibial bone.

In detail, the lower surface 201 is positioned above the tibial bone exposed during the implantation and in contact with it: the contact between the lower surface 201 of the tibial plate 20 and the tibial bone can occur directly, placing the tibial bone and the lower surface 201 in contact; alternatively and preferably, the contact with the tibial bone can occur indirectly, by interposing a quantity of medical cement between the exposed tibial bone and the lower surface 201 of the tibial plate 20.

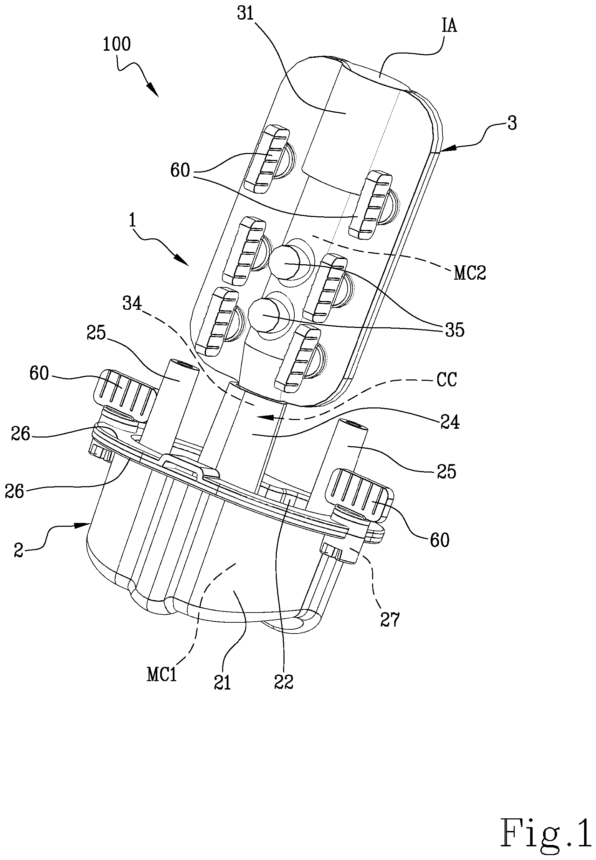

According to the preferred, non-limiting embodiment of the device 1, illustrated in Figures 1 to 2, the tibial plate module 2 comprises a first tibial plate half-module 21 and a second tibial plate half-module 22, which can be coupled to the first tibial plate half-module 21, to define the first moulding chamber MC1. For example, but not exclusively, the first tibial pate half-module 21 can locate the upper surface, for making tibial condyles and intercondylar eminence, and the lateral surface, whilst the second tibial plate half-module 22 can locate the lower surface of the first moulding chamber MC1. The first half-module 21 can therefore be used to make the lateral tibial condyle and the medial tibial condyle of the tibial plate 20 and the second half-module 22 can be used to make the lower surface 201 of the tibial plate 20.

Preferably, the first tibial plate half-module 21 and the second tibial plate half-module 22 can be coupled by means of respective flanges 26: in other words, the first and the second tibial plate half-modules 21, 22 comprise respective flanges 26 prepared to allow the mutual moving towards each other of the first and second tibial plate half-modules 21, 22 and to reach the desired mutual position and orientation between the first and second tibial plate half-modules 21, 22, so as to correctly define the first moulding chamber MC1.

The flanges 26 are positioned on the perimeter of the respective half-module 21, 22, whilst the above-mentioned proximal and distal surfaces of the first chamber MC1 are made centrally to the respective half-module 21, 22 so that, after the contact of the flanges 26 the first chamber MC1 is defined inside the closed tibial plate module 21.

Moreover, the first and second tibial plate half-modules 21, 22 may have one or more holes 27 at the flanges 26, said holes 27 being distributed in such a way as to be next to and coaxial with the correct coupling between the first and second tibia plate half-modules 21, 22. Said holes 27 are engaged in use by fixing means 60 to keep coupled the first and the second tibia plate half-modules 21, 22 during the injection of the liquid medical cement and its solidification.

As mentioned, the device 1 according to this invention also comprises the stem module 3. The stem module 3 defines a second moulding chamber MC2 for making a tibial stem 30 and may have an injection opening IA, configured in use for injecting liquid medical cement. More in detail, the second chamber MC2 is an inner space of this module 3 and is defined by the shape of a relative inner surface. The injection opening has a cylindrical shape or, in any case, with a substantially circular transversal cross section and can be coupled to a dispensing spout of a device for dispensing liquid medical cement of the type known to the sector.

The second moulding chamber MC2 is, in effect, configured for receiving a quantity of medical cement in a liquid form and for allowing the solidification, to obtain at least a part of or an entire tibial stem 30, which in use is configured for inserting inside the proximal portion of the tibial channel of a patient

More in detail, the second moulding chamber MC2 is elongated, that is to say, it has a main extension along a specific direction Y.

Consequently, the tibial stem 30 made in the second moulding chamber MC2 has an elongated shape along the direction Y (Figure 6, Figure 7). The tibial stem 30, thus shaped, is in use positioned at least at a portion of the tibial channel of the patient.

Considering a tibial diaphysis, that is, the portion of tibia with an elongated shape, which extends between the proximal epiphysis, articulated with the femur, and the distal epiphysis, articulated with the ankle, the term tibial channel means the tibial intramedullary channel, that is, the innermost portion of the tibial diaphysis. The intramedullary channel, like the tibial diaphysis, has an elongated extension between the proximal epiphysis and the distal epiphysis of the tibia and consists mainly of spongy bone, which is highly porous, bone marrow and blood vessels. The tibial intramedullary channel is delimited by more compact and resistant cortical bone, which constitutes the outermost layers of tibial bone.

In use, the tibial stem 30 is inserted inside the tibial intramedullary channel, so, once positioned, the tibial stem 30 occupies at least one upper portion of said tibial intramedullary channel.

In order to obtain a correct and stable positioning of the tibial spacer 10 at the tibia, an operator proceeds by tucking the tibial stem 30 inside the tibial intramedullary channel, preferably interposing a quantity of medical cement. Once correctly positioned, therefore, the tibial stem 30 of the femoral spacer 10 engages with at least one stretch of the tibial intramedullary channel, while the tibial plate 20 is positioned in contact with the upper portion of the tibial bone.

In the description below, the "tibial intramedullary channel" will be called, simplifying it, the "tibial channel".

According to a first embodiment, illustrated in Figures 1 to 3, the second moulding chamber MC2 has a substantially cylindrical shape and may have a constant or variable diameter.

The second moulding chamber MC2 shaped in this way allows a spacer 10 to be obtained having a tibial stem 30 with an elongated, and preferably substantially cylindrical, shape (see Figure 6) which may have a constant or variable diameter.

According to an alternative embodiment, not shown in the accompanying drawings, the second moulding chamber MC2 has a first stretch, or proximal stretch, and a second stretch, or distal stretch, which are parallel with the direction Y and offset from each other, said proximal stretch and said distal stretch being preferably connected by an intermediate connecting stretch. Preferably, said proximal stretch and said distal stretch of the second moulding chamber MC2 are cylindrical and connected by the intermediate connecting stretch, which may in practice be a substantially cylindrical connection. Again preferably, the proximal stretch, the distal stretch and the intermediate stretch have a substantially constant transversal cross-section. The second moulding chamber MC2 according to this latter embodiment allows a spacer 10 to be obtained comprising a tibial stem 30 having overall extension in the direction Y and having a proximal portion 301 and a distal portion 302 elongated along respective axes A1, A2, said axes A1, A2 being substantially parallel to the direction Y (Figure 7); in other words, said proximal portion 301 and said distal portion 302 are offset and connected by an intermediate connecting portion 303, to define an offset" between the two portions 302, 303. Advantageously, the presence of the offset allows a tibial stem 30 to be made which can be introduced into tibial channels which are not perfectly rectilinear without the risk of damaging the implant or the tibia of the patient. In fact, the tibial channel is not always perfectly rectilinear and therefore said offset, suitably dimensioned, allows the insertion of the tibial stem to be facilitated taking into account the anatomical variability of the patients.

According to the preferred but not exclusive embodiment of the device 1, the stem module 3 comprises a first stem half-module 31 and a second stem half-module 32, which can be coupled to the first stem half-module 31 to define the second moulding chamber MC2; preferably, the first stem half-module31 and the second stem half-module 32 can be coupled by means of respective flanges 36: in other words, the first and the second stem half-modules 31, 32 comprise respective flanges 36 prepared to allow the mutual moving towards each other of the first and second stem half-modules 31, 32 and for reaching the desired mutual position and orientation between the first and second stem half-module 31, 32, in order to correctly define the second moulding chamber MC2.

Also in the case of the stem module 3, the flanges 36 are positioned on the perimeter of the respective half-module 31, 32, whilst the inner surfaces which delimit and define the second chamber MC2 are made centrally to the respective half-module so that, following the contact of the flanges 36 the second chamber is formed inside the closed stem module 31. Moreover, the first and second stem half-modules 31,32 may have one or more holes 37 at the flanges 36, said holes 37 being distributed in such a way as to be next to and coaxial with the correct coupling between the first and second stem half-modules 31,32. Said holes 37 are engaged in use by fixing means 60 to keep coupled the first and the second stem half-modules 31, 32 during the injection of the liquid medical cement and its solidification. Further, the stem module 3 may have one or more weaknesses 38, corresponding to portions of the stem module 3 defining localised narrowing of the second moulding chamber MC2, designed to indicate and define preferred failure stretches of the device 1 at the stem module 3. The weaknesses 38 are made at the second moulding chamber MC2 and preferably define a plane perpendicular to the direction Y. In other words, each weakness 38 corresponds to an annular structure of the second moulding chamber MC2.

After the injection of medical cement in the device 1 through the injection opening IA and before the complete solidification of the medical cement, the stem module 3 can be broken at a weakness 38, so as to obtain a tibial spacer 10 having the tibial stem 30 of the desired length: the broken portion is in fact removed from the device 1, keeping the portion of stem module 3 closer to the tibial plate module 2.

Advantageously, the presence of a plurality of weaknesses 38 allows the device 1 to be used to obtain tibial spacers 10 having tibial stems 30 of different lengths.

According to a preferred version, the stem module 3 has at least two weaknesses 38: in detail, a first weakness 38 is closer to the end shank 34 and allows a tibial spacer 10 to be obtained having a tibial stem 30 with a shorter length; vice versa, a second weakness 30, more distal with respect to the end shank 34, allows a tibial spacer 10 to be obtained having a tibial stem 30 with a greater length.

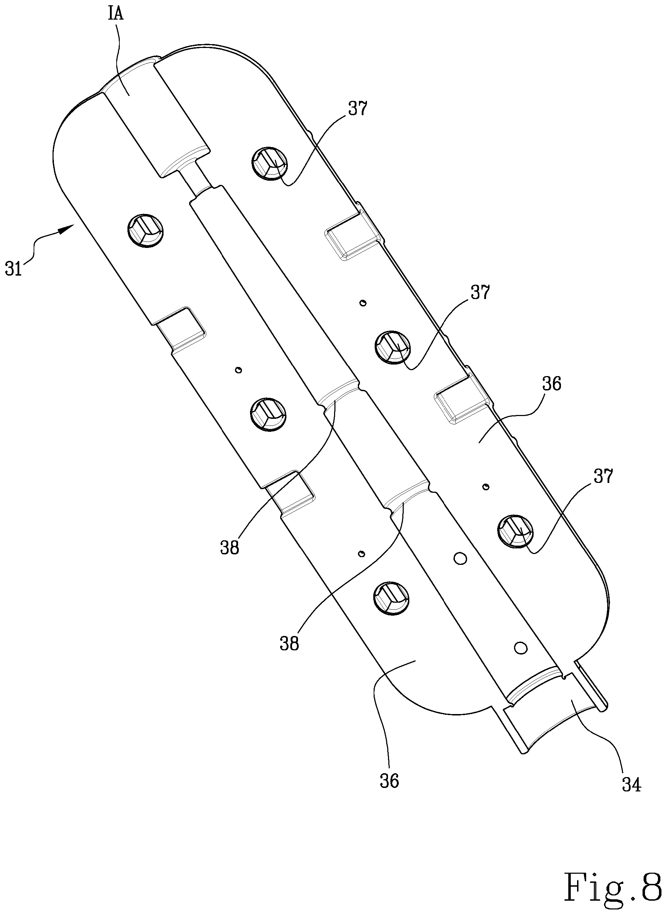

According to the version of the stem module 3 comprising the first stem half-module 31 and the second stem half-module 32, the weaknesses 38 are made on at least one between the first and second stem half-modules 31, 32, preferably on both; Figure 8 shows a first stem half-module 31 having two respective weaknesses 38, which can be coupled to corresponding weaknesses 38 situated at a second stem half-module 32. By coupling the first and second stem half-modules 31, 32, pairs of corresponding weaknesses 38 identify the above-mentioned localised narrowing of the second moulding chamber MC2, allowing the facilitated breakage of the stem module 3 at said weaknesses 38.

As mentioned above, according to a preferred version of the invention, which can be implemented both according to the embodiment in which a spacer with a rectilinear stem is produced and in which a spacer with a stem provided with an offset is produced, there is a connecting channel CC which places in fluid-dynamic communication the two moulding chambers MC1, MC2, which is defined when the two modules 2, 3 are coupled or in any case connected.

In this case, the second moulding chamber MC2 and the connecting channel CC define together a forming chamber for the entire stem 30. More specifically, according to the version wherein the two modules 2, 3 are separate and reversibly coupled, the connecting channel CC is defined by the joining of a cylindrical neck 24, included in an attachment site 24, protruding towards the outside with the tibial plate module 2 and an end shank 34 which protrudes towards the outside with the stem module 3. For example, the cylindrical neck 24 is designed to receive the shank 34 and in any case they may both be cylindrical in shape. Alternatively, the end shank 34 of the stem module 3 is designed to receive the cylindrical neck 24 of the tibial plate module 2.

In more general terms, the stem module 3 may be coupled to the tibial plate module 2 at an attachment site 24, located at the tibial plate module 2, to define the connecting channel CC. Preferably, the stem module 3 can be coupled in a removable fashion to the tibial plate module 2 at the above-mentioned attachment site 24.

The device 1 according to this invention, thanks to the presence of the connecting channel CC between the first and second moulding chambers MC1, MC2, advantageously allows the tibial spacer 10, comprising the tibial plate 20 and the tibial stem 30, to be moulded in a single body: the connecting channel CC is in effect shaped to allow the passage of the liquid medical cement, introduced through the injection opening IA, from the second to the first moulding chamber MC2, MC1 and vice versa, filling both the first and the second moulding chambers MC1, MC2.

When coupling is correctly made between the tibial plate module 2 and the stem module 3, the second moulding chamber MC2 is elongated along the direction Y and protruding from the first moulding chamber MC1; in particular, the second moulding chamber MC2 protrudes from the lower surface of the first moulding chamber MC1. Preferably, the second moulding chamber MC2 is substantially perpendicular to the lower surface of the first moulding chamber MC1.

Moreover, the device 1 according to this invention comprises one or more tubular bodies 25, having a main extension away from the first moulding chamber MC1, to make said first moulding chamber MC1 in communication with the outside environment, allowing the escape of air and excess medical cement. Advantageously, thanks to the presence of the tubular bodies 25, the excess liquid medical cement inside the first and second moulding chambers MC1, MC2 can escape from the device 1; similarly, any air bubbles trapped can escape, limiting the formation of defects and discontinuities in the inner structure of the tibial spacer 10 once solidified; lastly, thanks to the tubular bodies 25 it is possible to make the process for cooling the medical cement to form the tibial spacer 10 considerably faster. This invention also relates to an assembly 100 for making a tibial spacer 10 (Figures 1-2).

The assembly 100 according to this invention comprises a moulding device 1 according to this invention and a reinforcing core 40, preferably metallic, with an elongated shape, extending in the direction Y between a first and a second end 41, 42; the reinforcing core 40 is shaped in such a way that it can be located, in use, at least partly inside the second moulding chamber MC2 of the device 1, making a reinforcing element of the tibial spacer 10 at the stem 30. In effect, the reinforcing core 40 is completely immersed in the liquid medical cement injected through the injection opening IA: once solidified, the cement stabilises the reinforcing core 40 inside the tibial spacer 10.

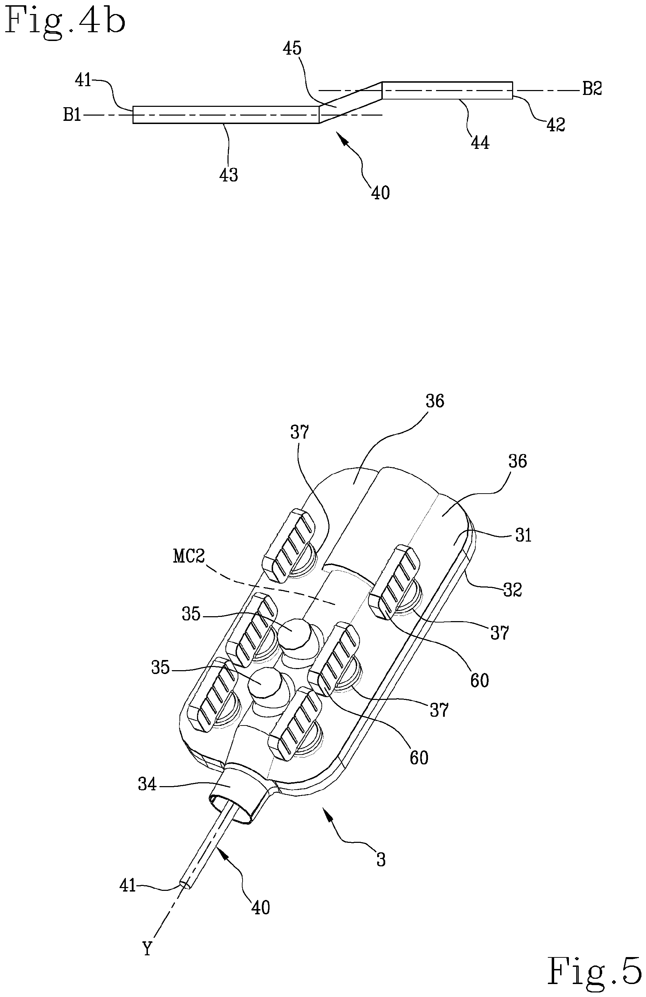

More in detail, the reinforcing core 40 is positioned inside the device 1 with the second end 42 inside the second moulding chamber MC2, and with the first end 41 inside the first moulding chamber MC1; in other words, the reinforcing core 40 partly escapes from the first moulding chamber (Figure 5), passes through the connecting channel CC and positions the first end 41 inside the first moulding chamber MC1.

Consequently, said reinforcing core 40 represents a reinforcing element at the interface between tibial plate 20 and tibial stem 30 in the tibial spacer 10.

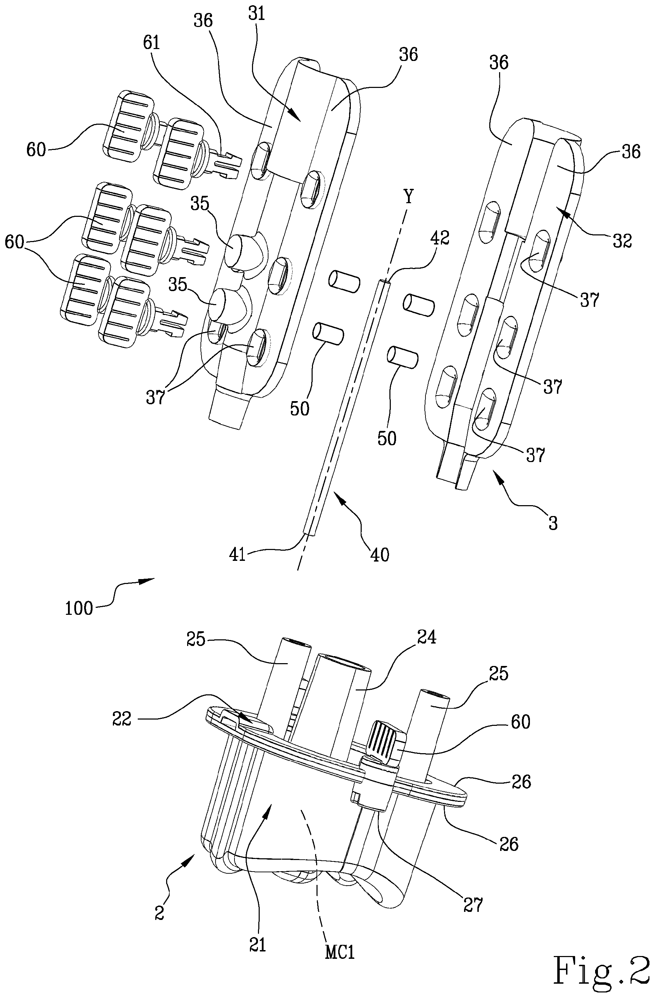

According to a first embodiment, illustrated in Figures 2 to 3, the reinforcing core 40 is rectilinear. Preferably, the reinforcing core 40 is substantially cylindrical in shape and extends in the direction Y. Moreover, the reinforcing core 40 is intended to be located inside a second rectilinear moulding chamber MC2, according to one of the embodiments described above. In this way, it is possible to obtain the tibial spacer 10 having a straight reinforced tibial stem 30 (Figure 6).

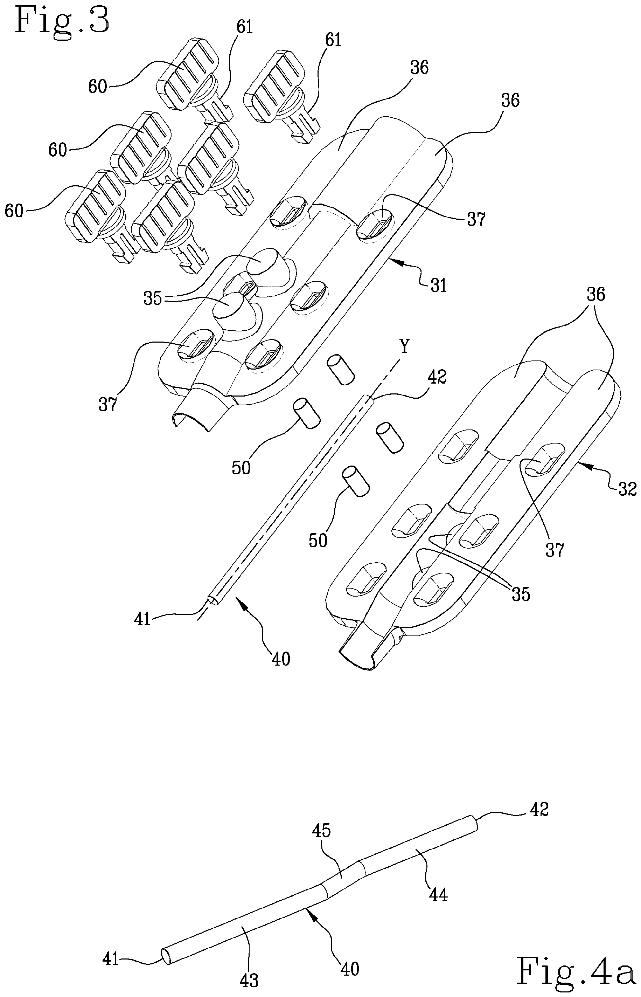

According to an alternative embodiment, illustrated in Figures 4a-4b, the core 40 has a proximal stretch 43 at the first end 41, and a distal stretch 44 at the second end 42, having axes B1, B2 parallel to the direction Y; more in detail, the proximal stretch 43 and the distal stretch 44 are offset from each other and connected by an intermediate connecting stretch 45. Preferably, the proximal stretch 43, the distal stretch 44 and the intermediate connecting stretch 45 have a substantially constant transversal cross section.

The reinforcing core 40 thus shaped is intended to be partly located inside the second moulding chamber MC2 having the proximal stretch and the distal stretch parallel to the direction Y and offset from each other, said proximal stretch and said distal stretch being connected by an intermediate connecting stretch: in particular, the proximal stretch 43, the intermediate connecting stretch 45 and the distal stretch 44 are inserted respectively in the proximal stretch, in the intermediate connecting stretch and in the distal stretch of the second moulding chamber MC2.

In this way, it is possible to obtain a tibial spacer 10 having a reinforced tibial stem 30 having the shape shown in Figure 7, said tibial stem 30 having the proximal portion 301 and the distal portion 302 parallel to the direction Y and offset and connected from the intermediate connecting portion 303, as shown in Figure 7.

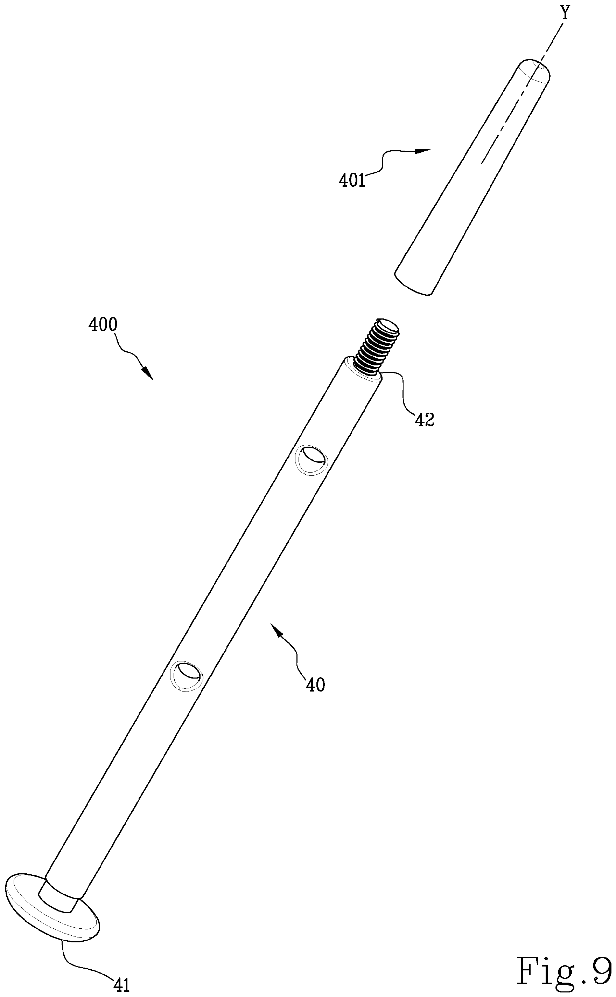

Further, the reinforcing core 40 may comprise an adapter element 401, which can be coupled to the reinforcing core 40 at its second end 42 (Figure 9); the adapter element 401 is elongate: preferably, the adapter element 401 is substantially cylindrical in shape.

The adapter element 401, through the coupling with the reinforcing core 40, advantageously allows a modular reinforcing core 400 to be obtained elongated parallel to the direction Y and effectively reinforcing tibial stems 30 having different lengths. In other words, the modular reinforcing core 400 has a reinforcing core 40 and adapter element 401 coupled and aligned parallel to the direction Y.

For example, the second end 42 may have a protruding threaded element, which can be coupled to a recessed peripheral portion of the adapter element 401: screwing the adapter element 401 to the core 40 obtains the modular reinforcing core 400.

The reinforcing core 40 may be used for reinforcing tibial stems 30 with smaller lengths; alternatively, the reinforcing core 40, coupled to the adapter element 401, may be used for reinforcing tibial stems 30 with greater lengths.

In other words, the assembly 100 according to this invention allows an operator to select just the reinforcing core 40 or to couple the adapter 401 to the reinforcing core 40, so as to adequately reinforce a tibial spacer 10 having the tibial stem 30 of a desired length. For this reason, the tibial spacers 10 having tibial stems 30 with greater lengths can also be adequately reinforced, avoiding the distal portions 302 from being less resistant and, therefore, more fragile than the proximal portions 301.

The assembly 100 according to this invention also comprises centring means 35, 50 for positioning the reinforcing core 40 in a predetermined position relative to at least the second moulding chamber MC2.

In detail, the centring means may comprise one or more seats 35 for centring the reinforcing core 40 made at the inner surface of the second moulding chamber MC2. More in detail, in order to keep the reinforcing core 40 correctly positioned inside the second moulding chamber MC2 and to prevent it from moving in an unpredictable and undesired manner during the injection of the liquid medical cement, the device 1 comprises, at the second moulding chamber MC2, one or more centring seats 35 made from blind holes transversely oriented relative to the direction Y and connected to the second moulding chamber MC2. The centring seats are preferably positioned in a specular manner between the first and second stem half-modules 31, 32, in such a way as to be facing each other and aligned. The centring means may comprise one or more centring pins 50 of elongated shape, designed to be inserted in the centring seats 35, prepared, in use, for maintaining the reinforcing core in the correct positioning. The centring pins 50 have an elongated shape, between a constraining end and a free end: the constraining end can be coupled to the reinforcing core 40 in such a way that said centring pin 50 is substantially perpendicular to the length of the reinforcing core 40, whilst the free end is shaped for inserting inside the blind holes making the centring seats 35. As many centring pins 50 may be coupled to the reinforcing core 50 perpendicularly to the direction Y as there are centring seats 35 in communication with the second moulding chamber MC2. In other words, the centring pins 50 of the assembly 100 according to this invention operate as means for anchoring the reinforcing core 40 to the device 1 and, in particular, to the stem module 3, in such a way that the reinforcing core 40 maintains the correct positioning and does not undergo undesired movements, for example, during injection of the liquid cement and before its complete solidification.

Lastly, the assembly 100 according to this invention comprises a plurality of fixing means 60. In order to keep the tibial plate half-modules 21, 22 and stem half-modules 31, 32 next to each other during the injection of the cement and to prevent unwanted relative movements between them, the holes 27, 37 made at the flanges 26, 36 may be advantageously engaged by the fixing means 60 included in the assembly according to this invention. The fixing means 60, in particular, operate between a constraining configuration, wherein they determine the coupling, respectively, of the first to the second tibial plate half-modules 21, 22 and of the first and second stem half-modules 31, 32, and a release configuration, wherein they determine the uncoupling of the first from the second tibial plate half-modules 21, 22 and of the first from the second stem half-modules 31, 32. Preferably, the fixing means 60 have a central reduction in area 61 designed to be broken in the passage from the constraining configuration to the release configuration. Fixing means operating as indicated are known to an expert in the trade.

This invention also relates to a kit for making in an operating room a plurality of tibial spacers 10 having different dimensions and three-dimensional configurations. In effect, tibial spacers 10 identical in shape and size may perform well for some patients and, on the other hand, may be not equally satisfactory for other patients, due to factors such as differences in height, age, state of health of the bone, and level of physical activity of said patients. In order to overcome this limitation, the kit according to this invention comprises a plurality of tibial plate modules 2, defining first moulding chambers MC1 having different dimensions and/or three-dimensional shapes, and a plurality of stem modules 3, defining second moulding chambers MC2 having different dimensions and/or three-dimensional shapes, said tibial plate modules 2 and said stem modules 3 being connectable to each other to define the connecting channel CC between the respective first and second moulding chambers MC1, MC2.

The tibial plate modules 2 may vary in size of the moulding chamber MC1, for example they may vary in thickness of said first moulding chamber MC1, to make tibial plates 20 of different dimensions within a range of interest.

The stem modules 3, on the other hand, may vary, for example, in terms of diameter and length of the second moulding chamber MC2, to make tibial stems 30 of different lengths, and/or for a three-dimensional configuration of the second moulding chamber MC2: in this second case, the operator can select, for example, between a stem module 3 making a substantially cylindrical tibial stem 30 or a tibial stem 30 with an offset, optionally also with different offsets, on the basis of data collected in the preoperative phase relative to the anatomy of the patient.

It is also possible that the kit comprises reinforcing cores 40 with different lengths and/or different thickness and/or offset.

Advantageously, the kit according to the invention allows the selection between a plurality of tibial spacers 10 which can be made, being particularly versatile and effective in responding to specific anatomical requirements. Advantageously, taking into account said specific anatomical requirements, the kit according to this invention allows the subsequent implant step to be facilitated, avoiding or, in any case, limiting undesired events, including the breakage of the stem due to impact with portions of cortical bone surrounding the tibial channel, or breakage of the bone following the insertion of a spacer with incorrect dimensions.

This invention also relates to a method for making a tibial spacer 10 in a single body comprising a tibial plate 20 and a stem 30 made of medical cement. It is possible to implement the proposed method using the moulding device 1 described above.

The method for making according to this invention comprises, firstly, the step of providing a first moulding chamber MC1 for making the tibial plate 20; in particular, said first moulding chamber MC1 may be identified by coupling the above-mentioned first and second tibial plate half-modules 21, 22 to form the tibial plate module 2.

Therefore, the method comprises the step of providing a second moulding chamber MC2 for making the stem 30; in particular, said second moulding chamber MC2 may be identified by coupling the above-mentioned first and second stem half-modules 31, 32 to form the stem module 3.

Considering the kit described above, the method according to the invention may comprise the preliminary step of selecting a tibial plate module 2 between the plurality of tibial plate modules 2 and a stem module between the plurality of stem modules 3.

Moreover, in order to make a tibial spacer 10 with a reinforcing core 40, before coupling the first stem half-module 31 to the second stem half-module 32, it is necessary to prepare one or more centring pins 50, coupling each pin to the reinforcing core 40, keeping said pin 50 substantially perpendicular to the direction Y, and, therefore, positioning each pin 50 coupled to the reinforcing core 50 inside a respective blind hole making a centring seat 35.

Further, it is necessary to position the reinforcing core 40 in such a way that, after coupling the first and second stem half-modules 3, it has the second end 42 inside the second moulding chamber MC2 and the first end 41 protruding from said second moulding chamber MC2 at the proximal portion 34.

The method also comprises the step of providing a connecting channel CC between the first and second moulding chambers MC1, MC2; in this step the stem module 3 is coupled to the tibial plate module 2 at the attachment site 24, defining a connecting channel CC at said attachment site 24.

In detail, the stem module 3, containing the reinforcing core 40 kept in position by the centring pins 50, is coupled to the tibial plate module 2, at the attachment site 24: the first end 41 of the reinforcing core 40 is inserted through the cylindrical neck 24 in the first moulding chamber MC1 until the above-mentioned shank 34 of the stem module 3 is inserted in the cylindrical neck 24. In this configuration, the tibial plate module 2 and the stem module 3 are reversibly coupled and define the connecting channel CC inside the neck 24.

Further, the method according to this invention comprises the step of preparing a quantity of medical cement in a liquid form; the medical cement in a liquid form is used in the subsequent step, which comprises the injection of said medical cement in a liquid form to fill the first moulding chamber MC1 and the second moulding chamber MC2. The injection of the medical cement occurs through a dispensing device of the known type to the sector and having a dispensing end. More in detail, the dispensing end is positioned at the injection opening IA of the stem module 3; the medical cement in liquid form is dispensed and flows through the second moulding chamber MC2 and the connecting channel CC, depositing in the first moulding chamber MC1 and, subsequently, in the second moulding chamber MC2, until filling. The dispensing device is then removed from the injection opening IA.

The method according to this invention also comprises the step of waiting for a predetermined time for the solidification of the medical cement inside the first and second moulding chambers MC1, MC2, forming in a single body the tibial spacer 10 inside said first and second moulding chambers MC1, MC2; the correct solidification is favoured by having tubular bodies 25.

It is necessary to remove said tubular bodies 25; preferably, the removal is performed by breaking, for example manually, said tubular bodies 25, applying a force directed perpendicularly to the direction Y.

Lastly, the method according to this invention comprises the step of releasing the tibial spacer 10 made of solidified medical cement; more specifically, in order to release the tibial spacer 10 it is necessary to uncouple the first from the second tibial plate half-modules 21, 22, and therefore the first from the second stem half-modules 31, 32.

If there are fixing means 60, before uncoupling the first and second tibial plate half-modules 21, 22 and stem half-modules 31, 32 it is necessary to move said fixing means 60 from the coupling configuration to the release configuration, for example, by breaking them at the central ridge 61.

The method according to this invention makes it possible to obtain in a fast and effective manner the tibial spacer 10 in a single body; in particular, the method according to the invention allows said tibial spacer 10 to be made in a single body whilst maintaining a constant production quality between subsequent operations, irrespective of the individual abilities of the operator and the particular intervention conditions.

The tibial spacer 10 according to the invention forms a tibial plate 20 and a stem 30 for a tibial channel. Inside the tibial spacer 10, the tibial plate 20 is in use set up to restore the functionality of the proximal tibial epiphysis, whilst the stem 30 is in use set up to be inserted inside the tibial channel of the patient, acting as a constraining and stabilising element for the tibial spacer 10.
the tibial plate 20 comprises an upper surface 202 opposite a lower surface 201, said upper surface 202 being connected to said lower surface 201 through a lateral surface defining the thickness of said tibial plate 20; the upper surface 202 is shaped to articulate with a corresponding femoral spacer to obtain a temporary knee prosthesis.

The tibial stem 30 projects from the tibial plate 20 at the lower surface 201, that is to say, opposite the articular surface 202.

The tibial spacer 10 according to this invention has a stem 30 having a proximal portion 301, constrained to the lower surface 201 of the tibial plate 20, and a distal portion 302, away from the lower surface 201.

As already explained above, according to a first embodiment, the proximal portion 301 and the distal portion 302 are aligned along the direction Y; therefore, the stem 30 has an elongated shape along said direction Y. Alternatively, the proximal portion 301 and the distal portion 302 may be parallel to the direction Y and offset from each other: according to this embodiment, the proximal portion 301 and the distal portion 302 are connected by an intermediate connecting portion 303, to make a tibial stem 30 with offset.

## Claims

1. A device (1) for making a tibial spacer (10) for a temporary knee prosthesis made of medical cement, comprising: a tibial plate module (2), defining a first moulding chamber (MC1) configured for making a tibial plate (20),
a stem module (3), defining a second moulding chamber (MC2) configured for making at least partly a tibial stem (30) suitable for the insertion in a tibial channel;
wherein the first moulding chamber (MC1) and the second moulding chamber (MC2) are fluid-dynamically communicating, to allow a tibial spacer (10) to be made in a single body.

2. The device according to claim 1, wherein the stem module (3) can be coupled in a removable fashion to the tibial plate module (2).

3. The device according to claim 2, wherein the stem module (3) can be coupled in a removable fashion to the tibial plate module (2) at an attachment site (24) made on the tibial plate module (2).

4. The device according to claim 3, wherein the attachment site (24) comprises a cylindrical neck (24) configured for receiving an end shank (34) of the stem module (3).

5. The device according to claim 3, wherein the stem module (3) comprises an end shank (34) configured for receiving a cylindrical neck included in the attachment site (24).

6. The device according to any preceding claim, wherein the first and second moulding chambers (MC1, MC2) communicate by means of a connecting channel (CC).

7. The device according to claim 4 or 5 and according to the preceding claim, wherein said connecting channel (CC) is defined by said cylindrical neck (24) and said end shank (34).

8. The device according to any preceding claim, wherein the second moulding chamber (MC2) has an elongated rectilinear shape.

9. The device according to any one of claims 1 to 5, wherein the second moulding chamber (MC2) has a first stretch and a second stretch offset relative to each other.

10. The device according to any preceding claim, wherein the tibial plate module (2) comprises a first tibial plate half-module (21) and a second tibial plate half-module (22), which can be coupled to the first tibial plate half-module (21) to define the first moulding chamber (MC1).

11. The device according to any preceding claim, wherein the tibial plate module (2) has one or more tubular bodies (25) having a main direction of extension away from the first moulding chamber (MC1) to make the first moulding chamber (MC1) in communication with the outside environment, allowing the escape of air and excess medical cement.

12. The device according to any preceding claim, wherein the stem module (3) comprises a first stem half-module (31) and a second stem half-module (32), which can be coupled to the first stem half-module (31) to define the second moulding chamber (MC2).

13. An assembly (100) for making a tibial spacer (10) of a temporary knee prosthesis comprising:
a device (1) according to any preceding claim and a reinforcing core (40) which can be positioned at least partly inside the second moulding chamber (MC2) of the device (1).

14. The assembly according to the preceding claim, wherein said reinforcing core (40) is elongated and rectilinear.

15. The assembly according to claim 13, wherein said reinforcing core (40) has a first stretch (43) and a second stretch (44) which are offset.

16. The assembly according to claim 11, wherein the device (1) comprises centring means (35, 50) for positioning the reinforcing core (40) in a predetermined position relative to the second moulding chamber (MC2).

17. The assembly according to the preceding claim, wherein said centring means comprise one or more centring seats (35) made from blind holes made in the second moulding chamber (MC2).

18. The assembly according to claim 16 or 17, wherein the centring means comprise one or more centring pins (50) which can be coupled to the reinforcing core (40) and designed to be inserted in respective centring seats.

19. A kit for making a plurality of tibial spacers (10) in an operating room in a single body, comprising:
a plurality of tibial plate modules (2) of a device according to claim 2, defining first moulding chambers (MC1) of different dimensions and/or shapes;
a plurality of stem modules (3) of a device according to claim 2, defining second moulding chambers (MC2) of different dimensions and/or shapes;
said for tibial plate modules (2) and said stem modules (3) being connectable to allow a communication between the first and the second moulding chambers (MC1, MC2).

20. A method for making a tibial spacer (10) in a single body comprising a tibial plate (20) and a stem (30) made of medical cement, said making method comprising the steps of:
- Providing a first moulding chamber (MC1) for making the tibial plate (20);
- Providing a second moulding chamber (MC2) for making the stem (30);
- Providing a connecting channel (CC) between said first and second moulding chambers (MC1, MC2);
- Preparing a quantity of medical cement in liquid form;
- Injecting the medical cement in a liquid form to fill the first moulding chamber (MC1) and the second moulding chamber (MC2);
- Waiting for a predetermined time for the solidification of the medical cement inside the first and the second moulding chambers (MC1, MC2), thereby forming a tibial spacer (10) in a single body.

21. A tibial spacer (10) for a temporary knee prosthesis made of medical cement forming in a single body a tibial plate (20) and a stem (30) for a tibial channel.

22. The spacer according to the preceding claim,
wherein said tibial plate (20) comprises an upper surface (202), in use designed for articulating with a femoral spacer to make the temporary knee prosthesis, opposite a lower surface (201), said upper surface (202) being connected to said lower surface (201) through a lateral surface defining the thickness of said tibial plate (20); and
wherein said stem (30) protrudes from the lower surface (201) of the tibial plate (20) and has a proximal portion (301) constrained to the lower surface (201) of the tibial plate (20) and a distal portion (302) away from the tibial plate (20).

23. The tibial spacer according to the preceding claim, wherein the proximal portion (301) and the distal portion (302) of the stem (30) are offset from each other.

24. The spacer according to claim 22, wherein the proximal portion (301) and the distal portion (302) of the stem (30) are aligned with each other.
